**Europäisches Patentamt**

**European Patent Office**

**Office européen des brevets**

(19)

(11) Veröffentlichungsnummer : **0 231 482**
**B1**

(12) **EUROPÄISCHE PATENTSCHRIFT**

(45) Veröffentlichungstag der Patentschrift :
**15.03.89**

(51) Int. Cl.⁴ : **C 07 C129/12**

(21) Anmeldenummer : **86117019.9**

(22) Anmeldetag : **08.12.86**

(54) Verfahren zur Isolierung von Diarylguanidinen.

(30) Priorität : **18.12.85 DE 3544730**

(43) Veröffentlichungstag der Anmeldung :
**12.08.87 Patentblatt 87/33**

(45) Bekanntmachung des Hinweises auf die Patenterteilung : **15.03.89 Patentblatt 89/11**

(84) Benannte Vertragsstaaten :
**DE FR GB IT**

(56) Entgegenhaltungen :
**DE—A— 1 518 818**
**US—A— 1 884 509**
**US—A— 1 897 220**

(73) Patentinhaber : **BAYER AG**
**Konzernverwaltung RP Patentabteilung**
**D-5090 Leverkusen 1 Bayerwerk (DE)**

(72) Erfinder : **Nierth, Alfred, Dr.**
**Bussardweg 7**
**D-4047 Dormagen 1 (DE)**
Erfinder : **Scherhag, Bernhard Dr.**
**Elisabeth-Langgässer Strasse 2**
**D-5090 Leverkusen (DE)**
Erfinder : **Preuss, Reinhard, Dr.**
**Franz-Fassbeder-Strasse 7**
**D-4047 Dormagen 1 (DE)**
Erfinder : **Klose, Heinz, Dr.**
**Olpener Strasse 454**
**D-5000 Köln 91 (DE)**
Erfinder : **Sabia, Salvatore**
**Domstrasse 43-45**
**D-5000 Köln (DE)**

# EP 0 231 482 B1

**Beschreibung**

Die Erfindung betrifft ein Verfahren zur Isolierung von Diarylguanidinen von hoher Reinheit in guter Ausbeute.

Bei der kontinuierlichen technischen Herstellung von Diarylguanidinen aus Anilin, Toluidinen oder Xylidinen und Chlorcyan fällt das Reaktionprodukt in Form einer dunkel gefärbten Schmelze an, die aus dem Hydrochlorid des entsprechenden Diarylguanidins und unerwünschten und für die Weiterverarbeitung zum reinen Diarylguanidin störenden Nebenprodukten besteht (DE-AS 1 518 818).

Die Schmelze wurde bisher dadurch gereinigt, daß man sie in Wasser löste und aus der sauren Diarylguanidinhydrochloridlösung durch Zugabe von Alkalilauge das freie Diarylguanidin ausfällte.

Das so gewonnene Diarylguanidin ist aber gefärbt und neigt zum Verschmieren, da Reste von nicht umgesetztem Amin und die bei der Synthese entstandenen Nebenprodukte mit ausgefällt werden.

Zur weiteren Reinigung wird die Diarylguanidinhydrochloridlösung mit Aktivkohle versetzt und nach entsprechender Einwirkungsdauer filtriert (US-PS 1 727 060). Ein solches Verfahren ist zwar wirksam aber unwirtschaftlich.

Andererseits ist bekannt (US-PS 1 897 220 ; US-PS 1 884 509), die wäßrige Lösung der Diarylguanidine mit wasserunlöslichen, unpolaren Kohlenwasserstoffen zu behandeln, so daß teilweise die hydrophoben Bestandteile der Verunreinigungen entfernt werden können. Nicht entfernt werden können jedoch alle aminischen Verunreinigungen, die in einer Diarylguanidinhydrochloridlösung ebenfalls als Hydrochloride vorliegen und in unpolaren Lösungsmitteln unlöslich sind.

Die Verwendung hydrophiler Lösungsmittel verbietet sich im allgemeinen wegen ihrer beträchtlichen Löslichkeit in der wäßrigen Guanidinhydrochloridlösung sowie umgekehrt wegen deren Lösung in dem entsprechenden Lösungsmittel. Das führt zu beträchtlichen Verlusten an Lösungsmittel wie auch an Guanidin.

Es wurde nun gefunden, daß man Diarylguanidine von hoher Reinheit und mit guten Ausbeuten aus rohen Diarylguanidin-hydrochloridschmelzen erhält, wenn man die heiße Schmelze in Wasser löst, diese Lösung mit einem Gemisch aus mindestens einem unpolaren, wasserunlöslichen Kohlenwasserstoff und mindestens einer polaren organishcen Verbindung extrahiert und aus der gereinigten wäßrigen Lösung durch Zugabe von Alkalihydroxid die reinen Diarylguanidine ausfällt.

Gegenstand der Erfindung ist also ein Verfahren zur Isolierung von Diarylguanidinen aus der Rohschmelze der Umsetzung von Arylaminen und Chlorcyan, dadurch gekennzeichnet, daß man die heiße Schmelze in Wasser löst, diese Lösung mit einem Gemisch aus mindestens einem wasserunlöslichen, unpolaren Kohlenwasserstoff aus der Reihe aliphatische, cycloaliphatische und aromatische Kohlenwasserstoffe mit einem Siedepunkt zwischen 80 und 200 °C und mindestens einer polaren organischen Verbindung aus der Reihe aliphatische und cycloaliphatische Alkohole mit 3 bis 10 Kohlenstoffatomen oder aromatische Nitrile in einem $p_H$-Bereich von 5 bis 7 extrahiert und aus der gereinigten wäßrigen Lösung das Diarylguanidin durch Zugabe von Alkalihydroxid ausfällt.

Als wasserunlösliche unpolare Lösungsmittel eignen sich insbesondere Toluol, o-, m-, p-Xylol, Ethyl- und Isopropylbenzol.

Als polare Extraktionsmittel eignen sich neben aliphatischen und cycloaliphatischen Alkoholen mit 3 bis 10 Kohlenstoffatomen aromatische Nitrile wie Benzonitril und Tolunitril.

Erfindungsgemäß können aber auch Gemische aus wasserunlöslichen Kohlenwasserstoffen bzw. Gemische polarer Extraktionsmittel eingesetzt werden. Das Mischungsverhältnis von polarem zu unpolarem Lösungsmittel kann in einem weiten Bereich schwanken. Vorzugsweise enthält das Lösungsmittelgemisch 5-40 Gew.-% an polarer Verbindung, ganz besonders bevorzugt 5-20 Gew.-%.

Die Verwendung von $C_3$-$C_{10}$-Alkoholen mit Toluol oder Xylol hat den Vorteil, daß sich nach der Extraktion die Phasen glatt trennen.

Ein weiterer Vorteil des erfindungsgemäßen Verfahren ist, daß das Extraktionssystem dem Reinigungsbedürfnis der Guanidinhydrochlorid-Rohlösung angepaßt werden kann. So läßt sich bei konstanten Mengenströmen in der Extraktionsapparatur allein durch Variation des Verhältnisses von polarem zu unpolarem Lösungsmittel trotz schwankender Qualität der Rohlösung ein gleichmäßig reines Diarylguanidin erzeugen.

Das Verfahren kann chargenweise oder kontinuierlich durchgeführt werden. Bevorzugt wird eine kontinuierliche Betriebsweise, bei der das Lösungsmittelgemisch ebenfalls kontinuierlich von den extrahierten Nebenprodukten gereinigt und in den Prozeß zurückgeführt wird.

Das Verfahren wird vorzugsweise bei 20 bis 100 °C durchgeführt.

Das Gewichtsverhältnis von Rohschmelze zu Wasser beträgt vorzugsweise 1 : 3 bis 1 : 20, ganz besonders bevorzugt 1 : 4 bis 1 : 10.

Das Gewichtsverhältnis organischer Lösungsmittel zu Wasser beträgt vorzugsweise 1 : 5 bis 1 : 20, ganz besonders bevorzugt 1 : 7 bis 1 : 14.

## Beispiel 1

a) 50 g einer ca. 160 °C warmen Diphenylguanidinhydrochlorid-Rohschmelze aus der Umsetzung von Anilin und Chlorcyan (DE-AS 15 18 818) wurden in 280 g heißem Wasser eingerührt.

Es bildete sich eine braune wäßrige Lösung, die aber noch ungelöste Anteile enthielt.

Diese Lösung wurde unter Rühren auf 60 °C abgekühlt und mit 30 g einer Mischung aus 10 Gew.-% Hexanol-1 und 90 Gew.-% o-Xylol versetzt.

Nachdem mit Natronlauge ein pH-Wert von 6,4 eingestellt worden war, wurde der Rührer abgestellt.

Die Phasen trennten sich sofort. Die wäßrige Phase wurde abgetrennt und nochmals mit 30 g der genannten Extraktionslösung bei pH 6,4 extrahiert.

Danach wurde durch Zugabe von Natronlauge auf pH 11,8 farbloses Diphenylguanidin ausgefällt. Es wurde mit Wasser gewaschen und getrocknet.

Die Ausbeute betrug 93,2 % der Theorie.

Die titrimetrische Analyse ergab einen Gehalt von 99,1 % und einen Schmelzpunkt 149,5 °C.

b) Als Vergleich wurden 50 g der selben Rohschmelze in 280 g heißem Wasser gelöst. Aus der Lösung wurde durch Zugabe von Natronlauge das Diphenylguanidin bei pH 11,8 ausgefällt.

Es entstand ein beigegefärbtes Produkt, das mit Wasser gewaschen und getrocknet wurde. Die Ausbeute betrug 98,3 % der Theorie.

| | |
|---|---|
| Wirkstoffgehalt (titrimetrisch) : | 98,1 % |
| Schmelzbereich : | 143,1-145,4 °C |
| Farbe : | Beige. |

## Beispiel 2

a) 50 g der warmen Rohschmelze wurden in 280 g heißem Wasser gelöst. Die wäßrige Lösung wurde abgekühlt und zweimal mit 20 g eines Gemischs aus 10 Gew.-% Benzonitril und 90 Gew.-% Toluol extrahiert.

Der pH wurde mit Natronlauge auf 6,2 eingestellt.

Nach Trennung der Phasen wurde die wäßrige Lösung wasserdampfdestilliert und danach das Diphenylguanidin durch Zugabe von Natronlauge ausgefällt.

Das Produkt war farblos und hatte einen Schmelzpunkt von 146,8 °C. Die Ausbeute betrug 94,1 % der Theorie.

## Beispiel 3

a) Bei einem kontinuierlich betriebenen Versuch wurde eine ca. 30 gew.-%ige wäßrige Roh-Diphenylguanidin-hydrochloridlösung durch Zugabe von Wasser auf 12 Gew.-% verdünnt und in einem Puffergefäß auf 60 °C temperiert. 50 l/h der verdünnten wäßrigen Lösung wurden dann im Gegenstrom mit 5 l/h eines Gemisches aus 95 Gew.-% o-Xylol und 5 Gew.-% Hexanol kontinuierlich extrahiert.

Durch gleichzeitige Zugabe von Natronlauge wurde der pH auf 6,9 eingestellt.

Der Versuch wurde in einer zweistufigen Mischer-Scheider-Batterie durchgeführt.

Die aus der Anlage abfließende wäßrige Lösung wurde in regelmäßigen Abständen analytisch untersucht.

| | |
|---|---|
| Schmelzpunkt : | 147,6 °C |
| Wirkstoffgehalt (titrimetrisch) : | 98,5 %. |

Die Ausbeute betrug 92,9 % der Theorie.

b) Parallel dazu wurde eine Probe aus der Roh-Diphenyl-guanidinhydrochloridlösung gezogen und deren Qualität durch Isolierung des Produkts durch Einrühren in Natronlauge bis pH 11,8 festgestellt. Der Schmelzbereich war 141,4-144,3 °C ; die Farbe des Produktes gelb. Die Ausbeute betrug 98,1 % der Theorie.

## Patentansprüche

1. Verfahren zur Isolierung von Diarylguanidinen aus der Rohschmelze der Umsetzung von Arylaminen und Chlorcyan, dadurch gekennzeichnet, daß man die heiße Schmelze in Wasser löst, diese Lösung mit einem Gemisch aus mindestens einem wasserunlöslichen, unpolaren Kohlenwasserstoff aus der Reihe aliphatische, cycloaliphatische und aromatische Kohlenwasserstoffe mit einem Siedepunkt zwischen 80 und 200 °C und mindestens einer polaren organischen Verbindung aus der Reihe aliphatische und cycloaliphatische Alkohole mit 3 bis 10 Kohlenstoffatomen oder aromatische Nitrile in einem $p_H$-Bereich von 5 bis 7 extrahiert und aus der gereinigten wäßrigen Lösung das Diarylguanidin durch Zugabe von Alkalihydroxid ausfällt.

2. Verfahren nach Anspruch 1, dadurch gekennzeichnet, daß die Extraktion bei Temperaturen zwischen 20 °C und 100 °C erfolgt.

3. Verfahren nach Anspruch 1, dadurch gekennzeichnet, daß das Lösungsmittelgemisch 5 bis 40 Gew.-% an polarer Verbindung enthält.

4. Verfahren nach Anspruch 1, dadurch gekennzeichnet, daß das Gewichtsverhältnis Rohschmelze zu Wasser 1 : 3 bis 1 : 20 beträgt.

5. Verfahren nach Anspruch 1, dadurch gekennzeichnet, daß das Gewichtsverhältnis organische Lösungsmittel zu Wasser 1 : 5 bis 1 : 20 beträgt.

## Claims

1. A process for the isolation of diaryl guanidines from the crude melt in the reaction of aryl amines and cyanogen chloride, characterized in that the hot melt is dissolved in water, the resulting solution is extracted with a mixture of at least one water-insoluble, apolar hydrocarbon from the series of aliphatic, cycloaliphatic and aromatic hydrocarbons having a boiling point of 80 to 200 °C and at least one polar organic compound from the series of aliphatic and cycloaliphatic alcohols containing 3 to 10 carbon atoms or aromatic nitriles at a pH in the range from 5 to 7 and the diaryl guanidine is precipitated from the purified aqueous solution by addition of alkali hydroxide.

2. A process as claimed in claim 1, characterized in that the extraction is carried out at temperatures of from 20 °C to 100 °C.

3. A process as claimed in claim 1, characterized in that the solvent mixture contains from 5 to 40 % by weight of polar compound.

4. A process as claimed in claim 1, characterized in that the ratio by weight of crude melt to water is from 1 : 3 to 1 : 20.

5. A process as claimed in claim 1, characterized in that the ratio by weight of organic solvent to water is from 1 : 5 to 1 : 20.

## Revendications

1. Procédé pour isoler des diarylguanidines de la masse fondue brute de réaction d'arylamines et de chlorure de cyanogène, caractérisé en ce qu'on dissout dans l'eau la masse fondue chaude, on extrait cette solution avec un mélange d'au moins un hydrocarbure non polaire insoluble dans l'eau choisi dans la série des hydrocarbures aliphatiques, cycloaliphatiques et aromatiques ayant un point d'ébullition compris entre 80 et 200 °C, et d'au moins un composé organique polaire choisi dans la série des alcools aliphatiques et cycloaliphatiques de 3 à 10 atomes de carbone ou de nitriles aromatiques dans un intervalle de pH de 5 à 7·et on précipite la diarylguanidine dans la solution aqueuse purifiée, par addition d'hydroxyde alcalin.

2. Procédé suivant la revendication 1, caractérisé en ce que l'extrait est effectué à des températures comprises entre 20 °C et 100 °C.

3. Procédé suivant la revendication 1, caractérisé en ce que le mélange de solvants contient 5 à 40 % en poids de composé polaire.

4. Procédé suivant la revendication 1, caractérisé en ce que le rapport en poids de la masse fondue brute à l'eau a une valeur de 1 : 3 à 1 : 20.

5. Procédé suivant la revendication 1, caractérisé en ce que le rapport en poids des solvants organiques à l'eau se situe entre 1 : 5 et 1 : 20.